(19) Europäisches Patentamt
European Patent Office
Office européen des brevets

(11) Veröffentlichungsnummer: **0 243 734 B1**

(12) **EUROPÄISCHE PATENTSCHRIFT**

(45) Veröffentlichungstag der Patentschrift: **17.07.91**

(51) Int. Cl.5: **C07D 513/04**, C07D 498/04, //A61K31/425,(C07D513/04, 277:00,235:00),(C07D498/04, 263:00,235:00)

(21) Anmeldenummer: **87105057.1**

(22) Anmeldetag: **06.04.87**

# (54) Optisch aktive Hydantoine.

(30) Priorität: **19.04.86 DE 3613246**
**07.02.87 DE 3703871**

(43) Veröffentlichungstag der Anmeldung:
**04.11.87 Patentblatt 87/45**

(45) Bekanntmachung des Hinweises auf die Patenterteilung:
**17.07.91 Patentblatt 91/29**

(84) Benannte Vertragsstaaten:
**CH DE FR GB IT LI**

(56) Entgegenhaltungen:
**AT-A- 341 525**
**DE-A- 2 425 306**
**US-A- 4 009 172**

**CHEMICAL ABSTRACTS Band 105, Nr. 13, 29. September 1986, Seite 674, Zusammenfassungsnr. 115004h, Columbus, Ohio, USA; A.V. EREMEEV et al.: "Reaction of 1,3-thiazolidine-4- and 1,4-tetrahydrothiazine-3-carboxylate esters with isocyanates and isothiocyanates and structure the reaction products"**

**CHIMIA 41, Nr. 5, Mai 1987, Seiten 148-150; E. POETSCH et al.: "Stereoselektive Synthesewege zu (+)-Biotin aus L-Cystein"**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Poetsch, Eike, Dr.**
**Am Buchwald 4**
**W-6109 Mühltal 6(DE)**
Erfinder: **Casutt, Michael, Dr.**
**Adolf-Kolping-Strasse 19**
**W-6102 Pfungstadt(DE)**

**EP 0 243 734 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue, optisch aktive Hydantoine der Formel I:

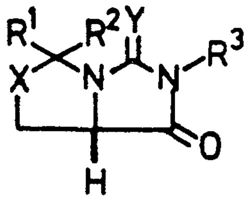

I

Hydantoine der Formel I sind wertvolle Ausgangsstoffe für die stereospezifische Synthese von D-(+)-Biotin.

Der Erfindung lag die Aufgabe zugrunde, neue Ausgangsstoffe bereitzustellen, die für die Herstellung optisch aktiven D-(+)-Biotins ohne Durchführung einer Racematspaltung und der damit verbundenen Notwendigkeit des Verwerfens oder Rückführens des unerwünschen Enantiomeren geeignet sind.

Verfahren zur stereospezifischen Synthese von D-(+)-Biotin aus Zuckern geeigneter Konfiguration sind bekannt. So wird in Tetrahedron Letters Nr. 32, S. 2765-2766 (1975), als Ausgangsmaterial D-Mannose, in Agric. Biol. Chem. Nr. 42, S. 465 (1978), D-Glucose und in den DE-OS 31 22 562 und DE-OS 33 20 140 D-Arabinose als chirales Ausgangsmaterial verwendet.

Alle diese Verfahren sind jedoch durch eine hohe Zahl von Syntheseschritten mit folglich geringer Gesamtausbeute gekennzeichnet. Die aufgrund ihrer Zuckernatur meist nicht kristallisierbaren Zwischenstufen werden oft nur in unbefriedigender Reinheit erhalten und erfordern, bedingt durch ihre Polyfunktionalität und der damit verbundenen chemischen Labilität, die Einhaltung vergleichsweise enger Reaktionsparameter. Eine Reihe von Zuckern ist auch aus natürlichen Quellen nicht zugänglich, was einen hohen Preis zur Folge hat.

Die Verwendung von L-Cystein, wie aus US-4009172, US-4130713, US-4337345 und Journal of the American Chemical Society Nr. 99, S. 7020 (1977) bekannt, vermeidet zwar die Handhabung labiler Zwischenstufen, führt hingegen über insgesamt 18 Reaktionsstufen unter Abtrennung unerwünschter Isomere nur in unbefriedigender Ausbeute zu optisch aktivem D-(+)-Biotin.

In einem weiteren Verfahren werden in Journal of the American Chemical Society Nr. 105, S. 5946 (1983) und in der EP-OS 0 094776 substituierte 3H,5H-Imidazo[1,5-c]tetrahydrothiazole beschrieben, aus denen nach Racematspaltung optisch aktives Biotin erhalten wird.

Da die vergleichsweise hohe Stufenzahl, verbunden mit teilweise mäßigen Ausbeuten und der Notwendigkeit einer optischen Trennung, auch diese Ausgangsstoffe als zu Herstellung von D-(+)-Biotin wenig geeignet erscheinen lassen, bestand weiterhin Bedarf an geeigneten Ausgangsstoffen zur einfachen, ökonomischen und stereospezifischen Herstellung von D-(+)-Biotin.

Aus der DE-A-24 25 306 und der AT-A 341-525 sind ähnliche, optisch aktive Hydantoine bekannt. Die dort beschriebenen Verbindungen sind Arzneistoffe, insbesondere Psychopharmaka und weisen im Gegensatz zu den erfindungsgemäßen Verbindungen anstelle der Reste $R^1$ und $R^2$ eine Spiropiperidin-4-yl Gruppe auf.

Von A.V. Eremeev et al. werden in Khim Geterosikl. Soedin 1985 (10), 1327-1332 (Chemical Abstracts, Band 105, Nr. 13, S. 674 Nr. 115004h) ähnliche, optisch aktive Hydantoine beschrieben, welche sich jedoch von erfindungsgemäßen durch die Gruppe $R^3$ unterscheiden. Es ist aber zur Lösung der erfindungsgemäßen Aufgabe, nämlich der Herstellung von optisch aktivem Biotin gemäß der EP-A-0242 686 unbedingt erforderlich, daß $R^3$ eine Amino-Schutzgruppe darstellt, welche nach chemischer Umwandlung des erfindungsgemäßen Zwischenproduktes leicht wieder abgespalten werden kann. Dies ist bei den bekannten Verbindungen nicht möglich.

Es wurde nun überraschend gefunden, daß sich die aus den natürlich vorkommenden, optisch aktiven Aminosäuren L-Cystein, L-Cystin und L-Serin erhältlichen kondensierten Hydantoine der Formel I zur Herstellung von D-(+)-Biotin auf stereospezifische Weise ohne zusätzliche Racemattrennung in hervorragender Weise eignen.

Gegenstand der Erfindung sind daher (7R)-1H,3H-Imidazo[1,5-c]azole der Formel I,

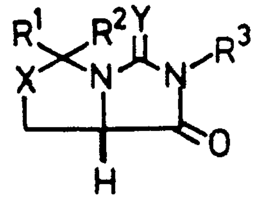

I

worin

X, Y jeweils unabhängig voneinander O oder S,

$R^1$, $R^2$ jeweils unabhängig voneinander H, $C_1$-$C_8$ Alkyl, worin auch eine oder zwei nicht benachbarte $CH_2$-Gruppen durch O oder S substituiert sein können, $C_3$-$C_8$ Cycloalkyl, unsubstituiertes oder durch $C_1$-$C_4$ Alkyl-und/oder $C_1$-$C_4$ Alkoxygruppen und/oder Halogenatome und/oder $C_1$-$C_4$ Alkoxycarbonylgruppen substituiertes $C_6$-$C_{14}$ Aryl und/oder $C_7$-$C_{12}$ Arylalkyl, $C_2$-$C_6$ Alk-1-en- oder Alk-2-enyl, zusammen $C_2$-$C_8$ Alkylen, worin auch eine oder zwei nicht benachbarte $CH_2$-Gruppen durch O oder S substituiert sein können,

$R^3$ Benzyl, durch eine oder mehrere $C_1$-$C_4$ Alkyl- und/oder $C_1$-$C_4$ Alkoxygruppen und/oder Halogenatome und/oder $C_1$-$C_4$ Alkoxycarbonylgruppen und/oder Nitrogruppen und/oder Cyanogruppen substituertes Benzyl, $C_2$-$C_6$ Alk-1-en-oder Alk-2-enyl, $C_2$-$C_6$ Alkoxyalkyl oder $C_3$-$C_9$ Trialkylsilyl bedeuten.

In dieser Formel bedeutet X vorzugsweise S und Y vorzugsweise O, insbesondere bevorzugt sind gleichzeitig X = S und Y = O. Die Reste $R^1$ und $R^2$ bedeuten vorzugsweise H, $C_1$-$C_4$-Alkyl, unsubstituiertes oder durch $C_1$-$C_3$ Alkyl und/oder Alkoxy ein- oder mehrfach substituiertes Phenyl oder Benzyl, insbesondere bevorzugt sind gleichzeitig $R^1$ = H, $R^2$ = Phenyl. Vorzugsweise bedeutet der Rest $R^3$ unsubstituiertes oder durch eine oder mehrere, vorzugsweise eine oder zwei, $C_1$-$C_4$ Alkyl- und/oder $C_1$-$C_4$-Alkoxygruppen substituiertes Benzyl, insbesondere bevorzugt unsubstituiertes Benzyl, daneben auch $C_3$-$C_5$ Alk-2-enyl oder $C_3$-$C_6$ Trialkylsilyl. Bei mehrfacher, vorzugsweise zweifacher, Substitution eines Phenylrings sind die Substituenten vorzugsweise gleich, sie können aber auch verschieden sein. Sie befinden sich vorzugsweise in 4- und/oder 2-Stellung, sie können aber auch in 3-, 5- und/oder 6-Stellung stehen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Gegenstand der Erfindung ist ferner ein Verfahren zur Herstellung der optisch aktiven Hydantoine der Formel I, dadurch gekennzeichnet, daß man L-Cystein oder L-Serin mit einem Alkali- oder Erdalkalimetallcyanat oder -thiocyanat in ein Hydantoin der Formel II umsetzt,

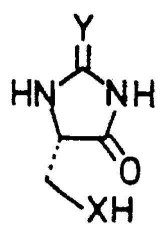

II

worin X und Y die angegebene Bedeutung besitzen, dieses mit einer Carbonylverbindung der Formel III,

$R^1$-CO-$R^2$ III

worin $R^1$ und $R^2$ die angegebene Bedeutung besitzen, unter Wasserabspaltung zu einem Bicyclus der Formel IV umsetzt,

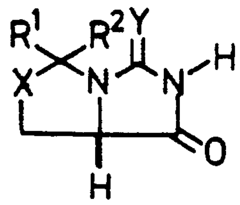

IV

worin $R^1$, $R^2$, X und Y die angegebene Bedeutung besitzen, und dessen sekundäres Stickstoffatom mit einer Schutzgruppe $R^3$ der angegebenen Bedeutung versieht,
oder daß man L-Cystin oder L-Serin mit einer Carbonylverbindung der Formel III unter Wasserabspaltung zu einem Azolidin V umsetzt,

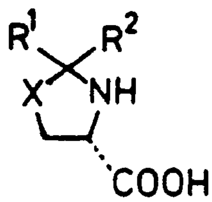

V

worin $R^1$, $R^2$ und X die angegebene Bedeutung besitzen, dieses mit einem Alkali- oder Erdalkalimetallcyanat oder -thiocyanat zu einer Verbindung der Formel IV umsetzt und weiter wie voranstehend angegeben verfährt, oder mit einem Organoisocyanat oder -isothiocyanat der Formel VI umsetzt,

$R^3$-N=C=Y VI

worin $R^3$ und Y die angegebene Bedeutung besitzen,
oder daß man L-Cystin mit einem Alkali- oder Erdalkalicyanat oder -thiocyanat zu einem Bishydantoin der Formel VII umsetzt,

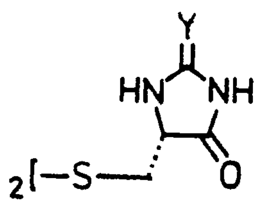

VII

worin Y O oder S bedeutet,
dieses durch ein Reduktionsmittel in ein Hydantoin der Formel II überführt und wie voranstehend angegeben weiter umsetzt, oder dessen in 3-Stellung befindliches Stickstoffatom mit einer Schutzgruppe $R^3$ der angegebenen Bedeutung versieht und das erhaltene Bishydantoin der Formel VIII,

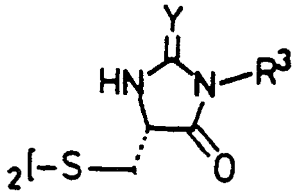

VIII

worin $R^3$ und Y die angegebene Bedeutung besitzen, nach Spaltung durch ein Reduktionsmittel mit einer Carbonylverbindung der Formel III umsetzt,
oder daß man L-Cystin mit einem Organoisocyanat oder -isothiocyanat der angegebenen Formel VI zu

einem Bishydantoin der Formel VIII umsetzt und wie voranstehend angegeben weiter verfährt.

Die Herstellung der Verbindungen der Formel I erfolgt im übrigen nach an sich bekannten Methoden, wie sie in der Literatur (z. B. in Standardwerken wie Houben-Weyl, Methoden der Organischen Chemie, Georg-Thieme-Verlag, Stuttgart) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formel II sind bekannt oder können nach bekannten Methoden aus L-Cystein oder L-Serin hergestellt werden wie beispielsweise in Schöberl, Hamm, Chem. Ber. 81 [1948], 210 und Karabinos, Szabo, J. Amer. Chem. Soc. 66 [1944], 649 angegeben, indem man die freien Aminosäuren oder deren Säureadditionssalze mit einem Alkali- oder Erdalkalimetallcyanat oder -thiocyanat in einem geeigneten Lösungsmittel wie Wasser, Alkoholen oder deren Gemischen, vorzugsweise bei erhöhter Temperatur, miteinander umsetzt und das erhaltene Zwischenprodukt in situ unter Einwirkung einer Säure, beispielsweise einer Mineralsäure, cyclisiert.

Die Umsetzung der Hydantoine der Formel II mit Carbonylverbindungen der Formel III zu den Bicyclen der Formel IV kann nach den für Acetalisierungen bekannten und gebräuchlichen Verfahren erfolgen, wie sie beispielsweise in Houben-Weyl, Methoden der Organischen Chemie, Bd. VI/3, S. 199, beschrieben sind. Vorzugsweise erfolgt die Umsetzung der Reaktionspartner unter Zusatz eines wasserentziehenden Mittels wie z. B. einer Säure wie Schwefelsäure, Phosphorsäure, Chlorwasserstoff, p-Toluolsulfonsäure, einem Säurederivat wie Phosphorpentoxid, Phosphortrichlorid, Phosphorpentachlorid, Phosphoroxychlorid, einem Metallsalz wie wasserfreiem Calciumchlorid, Kupfersulfat, Eisen(III)-chlorid, einem sauren Ionenaustauscher oder Molekularsieben. Die Entfernung des gebildeten Reaktionswassers kann auch durch azeotrope Destillation mit einem geeigneten Lösungsmittel wie Benzol, Toluol, Chloroform, Dichlormethan erfolgen. Schließlich ist es auch möglich, anstelle einer freien Carbonylverbindung der Formel III, deren Acetal mit einem geeigneten Alkohol, vorzugsweise einem niederen Alkohol, zur Herstellung der Verbindungen der Formel IV zu verwenden. Zweckmäßigerweise wird der bei der Reaktion freiwerdende Alkohol laufend aus dem Reaktionsgemisch entfernt, beispielsweise durch Destillation oder Adsorption. Auch kann durch einen Überschuß an Acetal von Oxoverbindung III gebildetes Reaktionswasser entfernt werden.

Für die Umsetzung mit den Hydantoinen der Formel II verwendet man vorteilhaft ein Äquivalent an Carbonylverbindung III bzw. deren Acetal, welche gleichzeitig als Lösungsmittel dienen können. Zweckmäßiger ist es jedoch, ein zusätzliches inertes Lösungsmittel zuzusetzen. Als inerte Lösungsmittel eignen sich vorzugsweise Kohlenwasserstoffe wie Pentan, Hexan, Cyclohexan, Benzol, Toluol oder Xylol sowie chlorierte Kohlenwasserstoffe wie Dichlormethan, Chloroform oder Tetrachlorkohlenstoff.

Zum Schutz des freien Stickstoffatoms in den Bicyclen der Formel IV werden diese mit einer reaktiven, den Rest $R^3$ mit der oben angegebenen Bedeutung tragenden Verbindung zu den (7R)-1H,3H-Imidazo[1,5-c]azolen der Formel I umgesetzt. Geeignete Verbindungen sind beispielsweise Benzylchlorid, Benzylbromid, 4-Methoxybenzylchlorid, 3,4-Dimethoxybenzylchlorid, 4-Methylbenzylchlorid, Benzyltosylat, Allylbromid, Methallylbromid, Crotylbromid, Chlordimethylether, Trimethylchlorsilan oder tert. Butyldimethylchlorsilan. Die Reaktionsbedingungen zur Einführung der Schutzgruppen entsprechen bekannten Verfahren wie sie beispielsweise aus Mac Omie, Protective Groups in Organic Chemistry, Plenum Press, New York, 1973 entnommen werden können.

Vorzugsweise erfolgt die Umsetzung der Reaktionspartner in einem geeigneten Lösungsmittel unter Zusatz eines basischen Reagenzes. Gut geeignete Lösungsmittel sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan.

Eine bevorzugte Reaktionsweise ist die Umsetzung einer bicyclischen Verbindung der Formel IV mit einer reaktiven, den Rest $R^3$ tragenden Verbindung, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natriumoder Kaliumacetat, Erdalkalimetallhydroxide wie Calciumhydroxid, Alkalimetallhydride wie Natriumhydrid, Amide wie Natriumamid, Lithiumdiisopropylamid, Alkoholate wie Natriummethylat, Natriumethylat, Lithiumethylat oder organische Basen wie Triethylamin, Pyridin, Lutidin, Collidin oder Chinolin von Bedeutung sind.

Die Reaktionstemperatur liegt gewöhnlich zwischen -50 °C und +250 °C, vorzugsweise zwischen -20 °C und +80 °C. Bei diesen Temperaturen sind die Reaktionen in der Regel nach 15 Minuten bis 48 Stunden beendet.

In einem weiteren Verfahren zur Herstellung der Verbindungen der Formel II setzt man L-Cystein oder

L-Serin mit einer Carbonylverbindung der Formel III zu einem Azolidin V um. Verbindungen der Formel V und Verfahren zu deren Herstellung sind beispielsweise bekannt aus Schubert, J. Biol. Chem. 114 (1936), 341, Uskovic et al., J. Amer. Chem. Soc. 97 (1975), 5936, Lieberman et al., ibid. 70 (1948), 3094 und US-3957794 sowie US-4009172. Ebenfalls geeignet sind die zur Herstellung der Verbindungen der Formel IV angegebenen Reaktionsbedingungen.

Azolidine der Formel V können mittels Alkalimetall- oder Erdalkalimetallcyanaten oder -thiocyanaten unter den bereits für die Herstellung von Verbindungen der Formel II angegebenen Reaktionsbedingungen in Verbindungen der Formel IV und weiter wie angegeben in die Imidazo[1,5-c] azole der Formel I überführt werden.

Zweckmäßiger ist es jedoch, die Azole der Formel V mit einem Organoisocyanat oder -isothiocyanat der Formel VI direkt zu den Imidazo[1,5-c]azolen der Formel I umzusetzen. Organoisocyanate und -isothiocyanate der Formel VI sind bekannt oder können nach bekannten Methoden wie beispielsweise in Houben-Weyl, Methoden der Organischen Chemie, Bde. VIII, S. 75 u. IX, S. 773, beschrieben, erhalten werden.

Aus Lieberman, J. Amer. Chem. Soc. 70 (1948), 3094 und Crabb et al., Tetrahedron 29 (1973), 3389, ist die Umsetzung von Hydantoinen mit Phenyl- und Methylisocyanat bekannt. Nach diesen Methoden kann auch die Reaktion der Azole der Formel V mit den Organoisocyanaten oder -isothiocyanaten der Formel VI erfolgen. Vorzugsweise wird die Umsetzung der Reaktionspartner in einem geeigneten Lösungsmittel wie z. B. Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan durchgeführt. Geeignet sind auch basische Lösungsmittel wie Pyridin, Lutidin, Collidin, Diethylamin oder Triethylamin sowie Gemische dieser Basen mit den voranstehend angegebenen Lösungsmitteln.

Gegebenenfalls kann es zweckmäßig sein, die primär gebildete Carbamoyl- oder Thiocarbamoylverbindung zu isolieren und in einem separaten Reaktionsschritt unter Wasserabspaltung zu cyclisieren. Als wasserabspaltende Mittel eignen sich beispielsweise Säuren wie Schwefelsäure, Chlorwasserstoff, Toluolsulfonsäure oder Basen wie Natrium- oder Kaliumhydroxid. Man kann dabei in Gegenwart oder Abwesenheit eines inerten Lösungsmittels bei Temperaturen zwischen etwa 0 °C und 150 °C, vorzugsweise zwischen etwa 20 °C und 100 °C, arbeiten; als Lösungsmittel kommen z. B. Wasser und Alkohole wie Methanol, Ethanol, Isopropanol oder Butanol in Betracht.

In einem weiteren Verfahren zur Herstellung der Verbindungen der Formel II setzt man L-Cystin mit einem Alkalimetall- oder Erdalkalimetallcyanat oder -thiocyanat zu einem Bishydantoin der Formel VII um. Dazu kann von den gleichen Methoden Gebrauch gemacht werden, die bereits zur Herstellung der Hydantoine der Formel II angegeben wurden, wobei gleiche oder ähnliche Reaktionsbedingungen zur Anwendung gelangen.

Aus den Bishydantoinen der Formel VII lassen sich durch Behandeln mit einem Reduktionsmittel Hydantoine der Formel II (X = S) erhalten, aus denen nach den voranstehend angegebenen Methoden die Imidazo[1,5-c]thiazole der Formel I (X = S) hergestellt werden. Geeignete Reduktionsmittel sind beispielsweise Houben-Weyl, Methoden der Organischen Chemie, Bd. 15/1, S. 798, zu entnehmen. Vorzugsweise eignen sich Natrium/flüssiger Ammoniak, Zink/Säure oder Phosphoniumiodid zur reduktiven Spaltung der Disulfid-Bindung. Zweckmäßigerweise führt man die Reduktion der Bishydantoine der Formel VII mit einem Äquivalent, insbesondere mit einem Überschuß Reduktionsmittel durch in einem geeigneten, der chemischen Natur des Reagenzes angepaßten Lösungsmittel wie z. B. Wasser, flüssigem Ammoniak, Alkoholen wie Methanol, Ethanol, Isopropanol, Säuren wie Salzsäure, Schwefelsäure, Ameisensäure, Essigsäure, Ethern wie Diethylether, THF oder Dioxan oder auch deren Gemischen, zweckmäßig bei Temperaturen zwischen etwa -50 °C und +150 °C.

Ein besonders vorteilhaftes Verfahren zur Spaltung der Disulfidbindung in den Verbindungen der Formel VII besteht in deren Thiolyse mit einem geeigneten Mercaptan wie z. B. Thiophenol, Butan-1,4-dithiol oder 1,4-Dithio-threitol analog zu der bei Hase, Walter, Inst. J. Pept. Prot. Res. 5 (1973), 283, angegebenen Arbeitsweise. Die Umsetzung der Reaktionspartner erfolgt zweckmäßigerweise in einem geeigneten Lösungsmittel wie z. B. wäßrigen Alkalimetallhydroxidlösungen, Chlorkohlenwassertoffen oder flüssigem Ammoniak bei Temperaturen zwischen etwa -40 °C und +120 °C.

Weiterhin können die Bishydantoine der Formel VII mit einer Schutzgruppe der Formel $R^3$ versehen werden. Zur Herstellung dieser geschützten Bishydantoine der Formel VIII kann von den bereits für die Herstellung der Verbindungen der Formel I aus den Bicyclen der Formel IV angegebenen Methoden Gebrauch gemavht werden, wobei gleiche oder ähnliche Reaktionsbedingungen zur Anwendung gelangen.

Ein weiteres Verfahren zur Herstellung der geschützten Bishydantoine der Formel VIII besteht in der

Umsetzung von L-Cystin mit einem Organoisocyanat oder -isothiocyanat der angegebenen Formel VI in Analogie zur Herstellung von Verbindungen der Formel I aus Azolidinen der Formel V, wobei man sich gleicher oder ähnlicher Verfahren bedient, wie bereits für die Herstellung der Verbindungen der Formel I angegeben.

Zur Überführung der Verbindungen der Formel VIII in die (7aR)-1H,3H-Imidazo[1,5-c]thiazole der Formel I (X = S) werden diese in einer der Herstellung der Hydantoine der Formel II aus den Disulfiden der Formel VII entsprechenden Weise unter Anwendung gleicher oder ähnlicher Verfahren und Reaktionsbedingungen mit einem Reduktionsmittel oder einem die Thiolyse bewirkenden Reagenz behandelt und darauf, entsprechend der Herstellung von Verbindungen der Formel V, mit einer Carbonylverbindung der Formel III umgesetzt.

Die erfindungsgemäßen (7aR)-1H,3H-Imidazo[1,5-c]azole der Formel I sind wertvolle Zwischenprodukte für die stereospezifische Herstellung von D-(+)-Biotin, wie beispielsweise in der gleichzeitig eingereichten DE-A1 36 13 246 angegeben.

Die folgenden Beispiele sollen die Erfindung erläutern, ohne sie zu begrenzen. Vor- und nachstehend bedeuten Prozentangaben Gewichtsprozent.

Beispiel 1

102,31 g (0,7 Mol) L-Cysteinhydantoin (Karabinos, Szabo, J. Am. Chem. Soc. 66 [1944], 649), werden in 1000 ml Toluol suspendiert und mit 74,28 g (0,7 Mol) Benzaldehyd versetzt.

Innerhalb von 30 Minuten tropft man bei 0 °C 107,33 g (0,7 Mol) Phosphorylchlorid zu, rührt weitere 12 Stunden bei Raumtemperatur, saugt vom Niederschlag ab und wäscht portionsweise mit 400 ml Toluol nach. Der Rückstand wird aus Methanol umkristallisiert. Man erhält 141,7 g (7aR)-3-Phenyl-1H,3H-imidazo-[1,5-c]thiazol-5,7(6H,7aH)-dion mit einem Schmelzpunkt von 177-179 °C.

$$[\alpha]^{20}_{365} = -1250°, \ c = 1 \ (\text{Methanol})$$

Analog werden hergestellt:
(7aR)-3-Methyl-1H,3H-imidazo[1,5-c]thiazol-5,7(6H,7aH)-dion
(7aR)-3-Ethyl-1H,3H-imidazo[1,5-c]thiazol-5,7(6H,7aH)-dion
(7aR)-3-Cyclopentyl-1H,3H-imidazo[1,5-c]thiazol-5,7(6H,7aH)-dion
(7aR)-3-Cyclohexyl-1H,3H-imidazo[1,5-c]thiazol-5,7(6H,7aH)-dion
(7aR)-3,3-Tetramethylen-1H,3H-imidazo[1,5-c]thiazol-5,7(6H,7aH)-dion
(7aR)-3,3-Pentamethylen-1H,3H-imidazo[1,5-c]thiazol-5,7(6H,7aH)-dion
(7aR)-3-(4-Methylphenyl)-1H,3H-imidazo[1,5-c]thiazol-5,7(6H,7aH)-dion
(7aR)-3-(4-Ethylphenyl)-1H,3H-imidazo[1,5-c]thiazol-5,7(6H,7aH)-dion
(7aR)-3-(4-Methoxyphenyl)-1H,3H-imidazo[1,5-c]thiazol-5,7(6H,7aH)-dion
(7aR)-3-(4-Ethoxyphenyl)-1H,3H-imidazo[1,5-c]thiazol-5,7(6H,7aH)-dion
(7aR)-3-Phenyl-1H,3H-imidazo[1,5-c]thiazol-5(6H)-thion-7(7aH)-on
(7aR)-3-Methyl-1H,3H-imidazo[1,5-c]thiazol-5(6H)-thion-7(7aH)-on
(7aR)-3-Ethyl-1H,3H-imidazo[1,5-c]thiazol-5(6H)-thion-7(7aH)-on
(7aR)-3-Cyclopentyl-1H,3H-imidazo[1,5-c]thiazol-5(6H)-thion-7(7aH)-on
(7aR)-3-Cyclohexyl-1H,3H-imidazo[1,5-c]thiazol-5(6H)-thion-7(7aH)-on
(7aR)-3,3-Tetramethylen-1H,3H-imidazo[1,5-c]thiazol-5(6H)-thion-7(7aH)-on
(7aR)-3,3-Pentamethylen-1H,3H-imidazo[1,5-c]thiazol-5(6H)-thion-7(7aH)-on
(7aR)-3-(4-Methylphenyl)-1H,3H-imidazo[1,5-c]thiazol-5(6H)-thion-7(7aH)-on
(7aR)-3-(4-Ethylphenyl)-1H,3H-imidazo[1,5-c]thiazol-5(6H)-thion-7(7aH)-on
(7aR)-3-(4-Methoxyphenyl)-1H,3H-imidazo[1,5-c]thiazol-5(6H)-thion-7(7aH)-on
(7aR)-3-(4-Ethoxyphenyl)-1H,3H-imidazo[1,5-c]thiazol-5(6H)-thion-7(7aH)-on
(7aR)-3-Phenyl-1H,3H-imidazo[1,5-c]oxazol-5,7(6H,7aH)-dion
(7aR)-3-Methyl-1H,3H-imidazo[1,5-c]oxazol-5,7(6H,7aH)-dion
(7aR)-3-Ethyl-1H,3H-imidazo[1,5-c]oxazol-5,7(6H,7aH)-dion
(7aR)-3-Cyclopentyl-1H,3H-imidazo[1,5-c]oxazol-5,7(6H,7aH)-dion
(7aR)-3-Cyclohexyl-1H,3H-imidazo[1,5-c]oxazol-5,7(6H,7aH)-dion
(7aR)-3,3-Tetramethylen-1H,3H-imidazo[1,5-c]oxazol-5,7(6H,7aH)-dion

(7aR)-3,3-Pentamethylen-1H,3H-imidazo[1,5-c]oxazol-5,7(6H,7aH)-dion
(7aR)-3-(4-Methylphenyl)-1H,3H-imidazo[1,5-c]oxazol-5,7(6H,7aH)-dion
(7aR)-3-(4-Ethylphenyl)-1H,3H-imidazo[1,5-c]oxazol-5,7(6H,7aH)-dion
(7aR)-3-(4-Methoxyphenyl)-1H,3H-imidazo[1,5-c]oxazol-5,7(6H,7aH)-dion
(7aR)-3-(4-Ethoxyphenyl)-1H,3H-imidazo[1,5-c]oxazol-5,7(6H,7aH)-dion

Beispiel 2

46,85 g (0,2 Mol) (7aR)-3-Phenyl-1H,3H-imidazo[1,5-c]thiazol-5,7(6H,7aH)-dion, gelöst in 450 ml Tetrahydrofuran, werden bei Raumtemperatur unter Stickstoff in eine Suspension von 6,6 g (0,22 Mol) 80%igem Natriumhydrid in 450 ml Tetrahydrofuran getropft. Nach ca. 30 Minuten ist die Wasserstoffentwicklung beendet. Nun tropft man 37,63 g (0,22 Mol) Benzylbromid in 15 Minuten zu, kocht 3 Stunden unter Rückfluß und destilliert das Lösungsmittel anschließend weitgehend ab.

Den Rückstand verteilt man zwischen 500 ml Dichlormethan und 500 ml Wasser, wäscht die organische Phase zweimal mit je 100 ml Wasser, trocknet über Natriumsulfat und engt unter vermindertem Druck ein.

Nach Verreiben mit Methanol erhält man 59,2 g (7aR)-3-Phenyl-6-benzyl-1H,3H-imidazo[1,5-c]thiazol-5,7(6H,7aH)-dion mit einem Schmelzpunkt von 78-79 °C.

$$[\alpha]_{365}^{20} = -1020°, \ c = 1 \ (\text{Methanol})$$

Analog werden hergestellt:
(7aR)-3-Phenyl-6-(4-methylphenyl)-1H,3H-imidazo[1,5-c]thiazol-5,7(6H,7aH)-dion
(7aR)-3-Phenyl-6-(4-methoxyphenyl)-1H,3H-imidazo[1,5-c]thiazol-5,7(6H,7aH)-dion
(7aR)-3-Phenyl-6-(4-chlorphenyl)-1H,3H-imidazo[1,5-c]thiazol-5,7(6H,7aH)-dion
(7aR)-3-Phenyl-6-(4-nitrophenyl)-1H,3H-imidazo[1,5-c]thiazol-5,7(6H,7aH)-dion
(7aR)-3-Phenyl-6-allyl-1H,3H-imidazo[1,5-c]thiazol-5,7(6H,7aH)-dion
(7aR)-3-Phenyl-6-trimethylsilyl-1H,3H-imidazo[1,5-c]thiazol-5,7(6H,7aH)-dion
(7aR)-3-Methyl-6-benzyl-1H,3H-imidazo[1,5-c]thiazol-5,7(6H,7aH)-dion
(7aR)-3-Methyl-6-(4-methylphenyl)-1H,3H-imidazo[1,5-c]thiazol-5,7(6H,7aH)-dion
(7aR)-3-Methyl-6-(4-methoxyphenyl)-1H,3H-imidazo[1,5-c]thiazol-5,7(6H,7aH)-dion
(7aR)-3-Methyl-6-(4-chlorphenyl)-1H,3H-imidazo[1,5-c]thiazol-5,7(6H,7aH)-dion
(7aR)-3-Methyl-6-(4-nitrophenyl)-1H,3H-imidazo[1,5-c]thiazol-5,7(6H,7aH)-dion
(7aR)-3-Methyl-6-allyl-1H,3H-imidazo[1,5-c]thiazol-5,7(6H,7aH)-dion
(7aR)-3-Methyl-6-trimethylsilyl-1H,3H-imidazo[1,5-c]thiazol-5,7(6H,7aH)-dion

Beispiel 3

Eine Lösung von 234,27 g (1 Mol) (7aR)-3-Phenyl-1H,3H-imidazo[1,5-c]thiazol-(6H,7aH)5,7-dion in 700 ml Dimethylformamid wird bei Raumtemperatur mit 138,19 g (1 Mol) Kaliumcarbonat und 130,39 g (1,03 Mol) Benzylchlorid versetzt und 3 Stunden bei 50 °C gerührt.

Anschließend wird filtriert und unter vermindertem Druck eingeengt.

Den öligen Rückstand rührt man in 950 ml Methanol ein und läßt bei 0 °C kristallisieren.

Man erhält 275,2 g (7aR)-3-Phenyl-6-benzyl-1H,3H-imidazo[1,5-c]thiazol-(6H,7aH)5,7-dion mit einem Schmelzpunkt von 77-78 °C.

$$[\alpha]_{365}^{20} = -1018°, \ c = 1 \ (\text{Methanol})$$

Analog werden erhalten:
(7aR)-3,3-Pentamethylen-6-benzyl-1H,3H-imidazo[1,5-c]thiazol-5,7(6H,7aH)-dion
(7aR)-3,3-Pentamethylen-6-(4-methylphenyl)-1H,3H-imidazo[1,5-c]thiazol-5,7(6H,7aH)-dion
(7aR)-3,3-Pentamethylen-6-(4-methoxyphenyl)-1H,3H-imidazo[1,5-c]thiazol-5,7(6H,7aH)-dion

(7aR)-3,3-Pentamethylen-6-(4-chlorphenyl)-1H,3H-imidazo[1,5-c]thiazol-5,7(6H,7aH)-dion
(7aR)-3,3-Pentamethylen-6-(4-nitrophenyl)-1H,3H-imidazo[1,5-c]thiazol-5,7(6H,7aH)-dion
(7aR)-3,3-Pentamethylen-6-allyl-1H,3H-imidazo[1,5-c]thiazol-5,7(6H,7aH)-dion
(7aR)-3,3-Pentamethylen-6-trimethylsilyl-1H,3H-imidazo[1,5-c]thiazol-5,7(6H,7aH)-dion
(7aR)-3-Phenyl-6-benzyl-1H,3H-imidazo[1,5-c]thiazol-5(6H)-thion-7(7aH)-on
(7aR)-3-Phenyl-6-(4-methylphenyl)-1H,3H-imidazo[1,5-c]thiazol-5(6H)-thion-7(7aH)-on
(7aR)-3-Phenyl-6-(4-methoxyphenyl)-1H,3H-imidazo[1,5-c]thiazol-5(6H)-thion-7(7aH)-on
(7aR)-3-Phenyl-6-(4-chlorphenyl)-1H,3H-imidazo[1,5-c]thiazol-5(6H)-thion-7(7aH)-on
(7aR)-3-Phenyl-6-(4-nitrophenyl)-1H,3H-imidazo[1,5-c]thiazol-5(6H)-thion-7(7aH)-on
(7aR)-3-Phenyl-6-allyl-1H,3H-imidazo[1,5-c]thiazol-5(6H)-thion-7(7aH)-on
(7aR)-3-Phenyl-6-trimethylsilyl-1H,3H-imidazo[1,5-c]thiazol-5(6H)-thion-7(7aH)-on

Beispiel 4

Nach der in Beispiel 3 angegebenen Arbeitsweise werden 234,27 g (1 Mol) (7aR)-3-Phenyl-1H,3H-imidazo[1,5-c]thiazol-(6H,7aH)5,7-dion mit 176,17 g (1,03 Mol) Benzylbromid und 138,19 g (1 Mol) Kaliumcarbonat in 700 ml Dimethylformamid zur Reaktion gebracht.

Man erhält 281,3 g (7aR)-3-Phenyl-6-benzyl-1H,3H-imidazo[1,5-c]thiazol-5,7-dion mit einem Schmelzpunkt von 77-78 °C.

$$[\alpha]_{365}^{20} = -1020°, \ c = 1 \ (\text{Methanol})$$

Analog werden hergestellt:
(7aR)-3-Methyl-6-benzyl-1H,3H-imidazo[1,5-c]thiazol-5(6H)-thion-7(7aH)-on
(7aR)-3-Methyl-6-(4-methylphenyl)-1H,3H-imidazo[1,5-c]thiazol-5(6H)-thion-7(7aH)-on
(7aR)-3-Methyl-6-(4-methoxyphenyl)-1H,3H-imidazo[1,5-c]thiazol-5(6H)-thion-7(7aH)-on
(7aR)-3-Methyl-6-(4-chlorphenyl)-1H,3H-imidazo[1,5-c]thiazol-5(6H)-thion-7(7aH)-on
(7aR)-3-Methyl-6-(4-nitrophenyl)-1H,3H-imidazo[1,5-c]thiazol-5(6H)-thion-7(7aH)-on
(7aR)-3-Methyl-6-allyl-1H,3H-imidazo[1,5-c]thiazol-5(6H)-thion-7(7aH)-on
(7aR)-3-Methyl-6-trimethylsilyl-1H,3H-imidazo[1,5-c]thiazol-5(6H)-thion-7(7aH)-on
(7aR)-3,3-Pentamethylen-6-benzyl-1H,3H-imidazo[1,5-c]thiazol-5(6H)-thion-7(7aH)-on
(7aR)-3,3-Pentmethylen-6-(4-methylphenyl)-1H,3H-imidazo[1,5-c]thiazol-5(6H)-thion-7(7aH)-on
(7aR)-3,3-Pentamethylen-6-(4-methoxyphenyl)-1H,3H-imidazo[1,5-c]thiazol-5(6H)-thion-7(7aH)-on
(7aR)-3,3-Pentamethylen-6-(4-chlorphenyl)-1H,3H-imidazo[1,5-c]thiazol-5(6H)-thion-7(7aH)-on
(7aR)-3,3-Pentamethylen-6-(4-nitrophenyl)-1H,3H-imidazo[1,5-c]thiazol-5(6H)-thion-7(7aH)-on
(7aR)-3,3-Pentamethylen-6-allyl-1H,3H-imidazo[1,5-c]thiazol-5(6H)-thion-7(7aH)-on
(7aR)-3,3-Pentamethylen-6-trimethylsilyl-1H,3H-imidazo[1,5-c]thiazol-5(6H)-thion-7(7aH)-on

Beispiel 5

Nach der in Beispiel 3 angegebenen Arbeitsweise werden 234,27 g (1 Mol) (7aR)-3-Phenyl-1H,3H-imidazo[1,5-c]thiazol-5,7-dion mit 130,39 g (1,03 Mol) Benzylchlorid, 138,19 g (1 Mol) Kaliumcarbonat und 14,99 g (0,1 Mol) Natriumjodid in 700 ml Dimethylformamid zur Reaktion gebracht.

Man erhält 288,9 g (7aR)-3-Phenyl-6-benzyl-1H,3H-imidazo[1,5-c]thiazol-5,7-dion mit einem Schmelzpunkt von 78-79 °C.

$$[\alpha]_{365}^{20} = -1023°, \ c = 1 \ (\text{Methanol})$$

Analog werden erhalten:
(7aR)-3-Methyl-6-benzyl-1H,3H-imidazo[1,5-c]oxazol-5,7(6H,7aH)-dion

(7aR)-3-Methyl-6-(4-methylphenyl)-1H,3H-imidazo[1,5-c]oxazol-5,7(6H,7aH)-dion
(7aR)-3-Methyl-6-(4-methoxyphenyl)-1H,3H-imidazo[1,5-c]oxazol-5,7(6H,7aH)-dion
(7aR)-3-Methyl-6-(4-chlorphenyl)-1H,3H-imidazo[1,5-c]oxazol-5,7(6H,7aH)-dion
(7aR)-3-Methyl-6-(4-nitrophenyl)-1H,3H-imidazo[1,5-c]oxazol-5,7(6H,7aH)-dion
(7aR)-3-Methyl-6-allyl-1H,3H-imidazo[1,5-c]oxazol-5,7(6H,7aH)-dion
(7aR)-3-Methyl-6-trimethylsilyl-1H,3H-imidazo[1,5-c]oxazol-5,7(6H,7aH)-dion
(7aR)-3-Phenyl-6-benzyl-1H,3H-imidazo[1,5-c]oxazol-5,7(6H,7aH)-dion
(7aR)-3-Phenyl-6-(4-methylphenyl)-1H,3H-imidazo[1,5-c]oxazol-5,7(6H,7aH)-dion
(7aR)-3-Phenyl-6-(4-methoxyphenyl)-1H,3H-imidazo[1,5-c]oxazol-5,7(6H,7aH)-dion
(7aR)-3-Phenyl-6-(4-chlorphenyl)-1H,3H-imidazo[1,5-c]oxazol-5,7(6H,7aH)-dion
(7aR)-3-Phenyl-6-(4-nitrophenyl)-1H,3H-imidazo[1,5-c]oxazol-5,7(6H,7aH)-dion
(7aR)-3-Phenyl-6-allyl-1H,3H-imidazo[1,5-c]oxazol-5,7(6H,7aH)-dion
(7aR)-3-Phenyl-6-trimethylsilyl-1H,3H-imidazo[1,5-c]oxazol-5,7(6H,7aH)-dion
(7aR)-3,3-Pentamethylen-6-benzyl-1H,3H-imidazo[1,5-c]oxazol-5,7(6H,7aH)-dion
(7aR)-3,3-Pentamethylen-6-(4-methylphenyl)-1H,3H-imidazo[1,5-c]oxazol-5,7(6H,7aH)-dion
(7aR)-3,3-Pentamethylen-6-(4-methoxyphenyl)-1H,3H-imidazo[1,5-c]oxazol-5,7(6H,7aH)-dion
(7aR)-3,3-Pentamethylen-6-(4-chlorphenyl)-1H,3H-imidazo[1,5-c]oxazol-5,7(6H,7aH)-dion
(7aR)-3,3-Pentamethylen-6-(4-nitrophenyl)-1H,3H-imidazo[1,5-c]oxazol-5,7(6H,7aH)-dion
(7aR)-3,3-Pentamethylen-6-allyl-1H,3H-imidazo[1,5-c]oxazol-5,7(6H,7aH)-dion
(7aR)-3,3-Pentamethylen-6-trimethylsilyl-1H,3H-imidazo[1,5-c]oxazol-5,7(6H,7aH)-dion

Beispiel 6

In einer unter Stickstoff stehenden Apparatur werden 20,93 g (0,1 Mol) (4S)-2-Phenyl-thiazolidincarbonsäure (Confalone et al., J. Amer. Chem. Soc. 99 (1977), 7020) in 200 ml Tetrahydrofuran vorgelegt. zu der Suspension tropft man innert 20 Minuten eine Lösung von 16,0 g (0,12 Mol) Benzylisocyanat in 50 ml Tetrahydrofuran und rührt das Gemisch eine Stunde bei 50 °C. Anschließend wird auf 0 °C abgekühlt, mit 30 ml konz. Salzsäure versetzt und 90 Minuten bei 60 °C gerührt.

Hiernach engt man ein, verteilt den Rückstand zwischen 200 ml Dichlormethan und 300 ml Wasser, wäscht die organische Phase zweimal mit je 100 ml Wasser, trocknet über Natriumsulfat und engt unter vermindertem Druck ein. Nach Verreiben mit Methanol erhält man 25,9 g (7aR)-3-Phenyl-6-benzyl-1H,3H-imidazo[1,5-c]thiazol-5,7(6H,7aH)-dion mit einem Schmelzpunkt von 79 °C.

$$[\alpha]_{365}^{20} = -1010°, \ c = 1 \ (\text{Methanol})$$

Analog werden erhalten:
(7aR)-3-Phenyl-6-(4-methylphenyl)-1H,3H-imidazo[1,5-c]thiazol-5,7(6H,7aH)-dion
(7aR)-3-Phenyl-6-(4-methoxyphenyl)-1H,3H-imidazo[1,5-c]thiazol-5,7(6H,7aH)-dion
(7aR)-3-Phenyl-6-(4-chlorphenyl)-1H,3H-imidazo[1,5-c]thiazol-5,7(6H,7aH)-dion
(7aR)-3-Phenyl-6-(4-nitrophenyl)-1H,3H-imidazo[1,5-c]thiazol-5,7(6H,7aH)-dion
(7aR)-3-Phenyl-6-allyl-1H,3H-imidazo[1,5-c]thiazol-5,7(6H,7aH)-dion
(7aR)-3-Phenyl-6-trimethylsilyl-1H,3H-imidazo[1,5-c]thiazol-5,7(6H,7aH)-dion
(7aR)-3-Methyl-6-benzyl-1H,3H-imidazo[1,5-c]thiazol-5,7(6H,7aH)-dion
(7aR)-3-Methyl-6-(4-methylphenyl)-1H,3H-imidazo[1,5-c]thiazol-5,7(6H,7aH)-dion
(7aR)-3-Methyl-6-(4-methoxyphenyl)-1H,3H-imidazo[1,5-c]thiazol-5,7(6H,7aH)-dion
(7aR)-3-Methyl-6-(4-chlorphenyl)-1H,3H-imidazo[1,5-c]thiazol-5,7(6H,7aH)-dion
(7aR)-3-Methyl-6-(4-nitrophenyl)-1H,3H-imidazo[1,5-c]thiazol-5,7(6H,7aH)-dion
(7aR)-3-Methyl-6-allyl-1H,3H-imidazo[1,5-c]thiazol-5,7(6H,7aH)-dion
(7aR)-3-Methyl-6-trimethylsilyl-1H,3H-imidazo[1,5-c]thiazol-5,7(6H,7aH)-dion
(7aR)-3,3-Pentamethylen-6-benzyl-1H,3H-imidazo[1,5-c]thiazol-5,7(6H,7aH)-dion
(7aR)-3,3-Pentamethylen-6-(4-methylphenyl)-1H,3H-imidazo-[1,5-c]thiazol-5,7(6H,7aH)-dion
(7aR)-3,3-Pentamethylen-6-(4-methoxyphenyl)-1H,3H-imidazo[1,5-c]thiazol-5,7(6H,7aH)-dion
(7aR)-3,3-Pentamethylen-6-(4-chlorphenyl)-1H,3H-imidazo[1,5-c]thiazol-5,7(6H,7aH)-dion
(7aR)-3,3-Pentamethylen-6-(4-nitrophenyl)-1H,3H-imidazo[1,5-c]thiazol-5,7(6H,7aH)-dion

(7aR)-3,3-Pentamethylen-6-allyl-1H,3H-imidazo[1,5-c]thiazol-5,7(6H,7aH)-dion
(7aR)-3,3-Pentamethylen-6-trimethylsilyl-1H,3H-imidazo[1,5-c]thiazol-5,7(6H,7aH)-dion

**Patentansprüche**

1. Optisch aktive Hydantoine der Formel I,

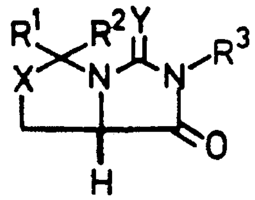

I

worin

X, Y jeweils unabhängig voneinander O oder S,

$R^1$, $R^2$ jeweils unabhängig voneinander H, $C_1$-$C_8$ Alkyl, worin auch eine oder zwei nicht benachbarte $CH_2$-Gruppen durch O oder S substituiert sein können, $C_3$-$C_8$ Cycloalkyl, unsubstituiertes oder durch $C_1$-$C_4$ Alkyl-und/oder $C_1$-$C_4$ Alkoxygruppen und/oder Halogenatome und/oder $C_1$-$C_4$-Alkoxycarbonylgruppen substituiertes $C_6$-$C_{14}$ Aryl und/oder $C_7$-$C_{12}$ Arylalkyl, $C_2$-$C_6$ Alk-1-en- oder Alk-2-enyl, zusammen $C_2$-$C_8$ Alkylen, worin auch eine oder zwei nicht benachbarte $CH_2$- Gruppen durch O oder S substituiert sein können,

$R^3$ Benzyl, durch eine oder mehrere $C_1$-$C_4$ Alkyl- und/oder $C_1$-$C_4$ Alkoxygruppen und/oder Halogenatome und/oder $C_1$-$C_4$ Alkoxycarbonylgruppen und/oder Nitrogruppen und/oder Cyanogruppen substituiertes Benzyl, $C_2$-$C_6$ Alk-1-en- oder Alk-2-enyl, $C_2$-$C_6$ Alkoxyalkyl oder $C_3$-$C_9$ Trialkylsilyl bedeuten.

2. Verfahren zur Herstellung von Hydantoinen der Formel I nach Anspruch 1, dadurch gekennzeichnet, daß man L-Cystein oder L-Serin mit einem Alkali-oder Erdalkalicyanat oder -thiocyanat in ein Hydantoin der Formel II umsetzt,

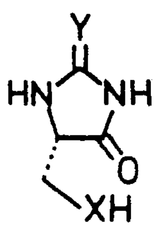

II

worin X und Y die angegebene Bedeutung besitzen, dieses mit einer Carbonylverbindung der Formel III,

$R^1$-CO-$R^2$ III

worin $R^1$ und $R^2$ die angegebene Bedeutung besitzen, unter Wasserabspaltung zu einem Bicyclus der Formel IV umsetzt,

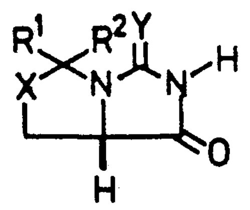

IV

worin $R^1$, $R^2$, X und Y die angegebene Bedeutung besitzen, und dessen sekundäres Stickstoffatom mit einer Schutzgruppe $R^3$ der angegebenen Bedeutung versieht,

oder daß man L-Cystein oder L-Serin mit einer Carbonylverbindung der Formel III unter Wasserabspaltung zu einem Azolidin V umsetzt,

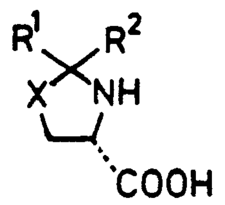

V

worin $R^1$, $R^2$ und X die angegebene Bedeutung besitzen, dieses mit einem Alkali- oder Erdalkalimetallcyanat oder -thiocyanat zu einer Verbindung der Formel IV umsetzt und weiter wie voranstehend angegeben verfährt, oder V mit einem Organoisocyanat oder -isothiocyanat der Formel VI umsetzt,

$R^3$-N=C=Y VI

worin $R^3$ und Y die angegebene Bedeutung besitzen,

oder daß man L-Cystin mit einem Alkali- oder Erdalkalicyanat oder -thiocyanat zu einem Bishydantoin der Formel VII umsetzt,

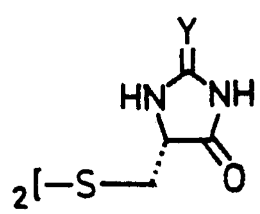

VII

worin Y O oder S bedeutet,

dieses durch ein Reduktionsmittel in ein Hydantoin der Formel II überführt und wie voranstehend angegeben weiter umsetzt, oder dessen in 3-Stellung befindliches Stickstoffatom mit einer Schutzgruppe $R^3$ der angegebenen Bedeutung versieht und das erhaltene Bishydantoin der Formel VIII,

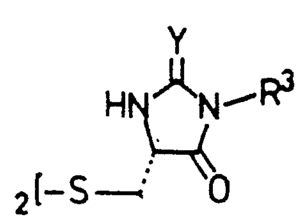

VIII

worin $R^3$ und Y die angegebene Bedeutung besitzen, nach Spaltung durch ein Reduktionsmittel mit einer Carbonylverbindung der Formel III umsetzt,

oder daß man L-Cystin mit einem Organoisocyanat oder -isothiocyanat der angegebenen Formel VI zu einem Bishydantoin der Formel VIII umsetzt und wie voranstehend angegeben weiter verfährt.

3. Verwendung von Hydantoinen der Formel I nach Anspruch 1 zur Herstellung von (D)-(+)-Biotin.

## Claims

1. Optically active hydantoins of the formula I

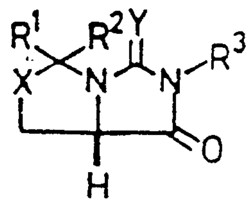

I

wherein

| | |
|---|---|
| X and Y | each independently of one another are O or S, |
| $R^1$ and $R^2$ | each independently of one another are H, $C_1$-$C_8$ alkyl, wherein one or two non-adjacent $CH_2$ groups can also be substituted by O or S, $C_3$-$C_8$ cycloalkyl, $C_6$-$C_{14}$ aryl and/or $C_7$-$C_{12}$ arylalkyl which is unsubstituted or substituted by $C_1$-$C_4$ alkyl and/or $C_1$-$C_4$ alkoxy groups and/or halogen atoms and/or $C_1$-$C_4$-alkoxycarbonyl groups, or $C_2$-$C_6$ alk-1-en- or alk-2-enyl, or together are $c_2$-$c_8$ alkylene, wherein one or two non-adjacent $CH_2$ groups can also be replaced by O or S, or |
| $R^3$ | is benzyl, benzyl which is substituted by one or more $C_1$-$C_4$ alkyl and/or $C_1$-$C_4$ alkoxy groups and/or halogen atoms and/or $C_1$-$C_4$ alkoxycarbonyl groups and/or nitro groups and/or cyano groups, $C_2$-$C_6$ alk-1-en- or alk-2-enyl, $C_2$-$C_6$ alkoxyalkyl or $C_3$-$C_9$ trialkylsilyl. |

2. Process for the preparation of hydantoins of the formula I according to Claim 1, characterised in that L-cysteine or L-serine is reacted with an alkali metal cyanate or thiocyanate or an alkaline earth metal cyanate or thiocyanate to give a hydantoin of the formula II

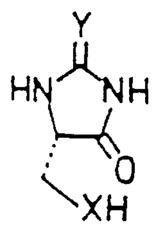

II

wherein X and Y have the meaning given, this is reacted with a carbonyl compound of the formula III

$R^1$-CO-$R^2$ III

wherein $R^1$ and $R^2$ have the meaning given, water being split off, to give a bicyclic compound of the formula IV

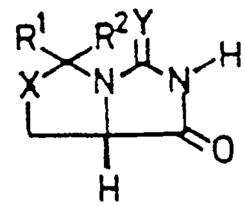

IV

wherein $R^1$, $R^2$, x and Y have the meaning given, and its secondary nitrogen atom is provided with a protective group $R^3$ of the meaning given, or in that L-cysteine or L-serine is reacted with a carbonyl compound of the formula III, water being split off, to give an azolidine V

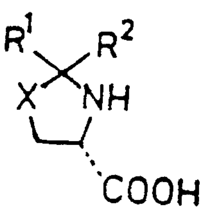

V

wherein $R^1$, $R^2$ and X have the meaning given, and this is reacted with an alkali metal cyanate or thiocyanate or an alkaline earth metal cyanate or thiocyanate to give a compound of the formula IV, and the subsequent procedure is as described above, or V is reacted with an organic isocyanate or isothiocyanate of the formula VI

$R^3-N=C=Y$ VI

wherein $R^3$ and Y have the meaning given, or in that L-cystine is reacted with an alkali metal cyanate or thiocyanate or an alkaline earth metal cyanate or thiocyanate to give a bishydantoin of the formula VII

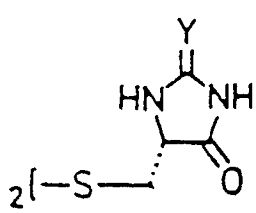

VII

wherein Y is O or S, this is converted into a hydantoin of the formula II by a reducing agent and the subsequent reaction is as described above, or its nitrogen atom in the 3-position is provided with a protective group $R^3$ with the meaning given, and the resulting bishydantoin of the formula VIII

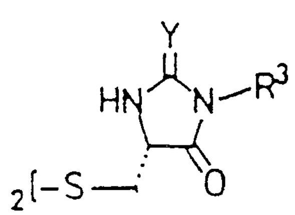

VIII

wherein $R^3$ and Y have the meaning given, after cleavage by a reducing agent, is reacted with a carbonyl compound of the formula III, or L-cystine is reacted with an organic isocyanate or isothiocyanate of the formula VI given to give a bishydantoin of the formula VIII and the subsequent

procedure is as described above.

3. Use of hydantoins of the formula I according to Claim 1 for the preparation of (D)-(+)-biotin.

## Revendications

1. Hydantoïnes optiquement actives de formule I

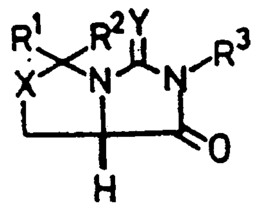

I

où

X, Y représentent indépendamment l'un de l'autre O ou S,

$R^1$, $R^2$ représentent indépendamment l'un de l'autre H, un alkyle en $C_1$-$C_8$, un ou deux groupes $CH_2$ non voisins pouvant être également substitués par O ou S, un cycloalkyle en $C_3$-$C_8$, un aryle en $C_6$-$C_{14}$ non substitué ou substitué par des groupes alkyles en $C_1$-$C_4$ et/ou par des groupes alcoxy en $C_1$-$C_4$ et/ou par des atomes d'halogène et/ou par des groupes alcoxy en $C_1$-$C_4$ carbonyles et/ou un arylalkyle en $C_7$-$C_{12}$, un alk-1-ényle en $C_2$-$C_6$ ou un alk-2-ényle en $C_2$-$C_6$, représentent ensemble un alkylène en $C_2$-$C_8$, un ou deux groupes $CH_2$ non voisins pouvant également être substitués par O ou S,

$R^3$ représente un benzyle, un benzyle substitué par un ou plusieurs groupes alkyles en $C_1$-$C_4$ et/ou groupes alcoxy en $C_1$-$C_4$ et/ou atomes d'halogène et/ou groupes alcoxy en $C_1$-$C_4$ carbonyle et/ou groupes nitro et/ou groupes cyano, un alk-1-ényle en $C_2$-$C_6$ ou un alk-2-ényle en $c_2$-$C_6$, un alcoxy en $C_2$-$C_6$ ou un trialkyle en $C_3$-$C_9$ silyle.

2. Procédé pour la préparation des hydantoïnes de formule I selon la revendication 1, caractérisé en ce que l'on fait réagir la L-cystéine ou la L-sérine avec un cyanate ou un thiocyanate d'un métal alcalin ou alcalino-terreux pour former une hydantoïne de formule II

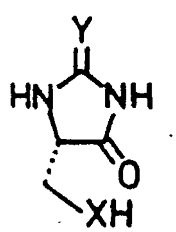

II

où X et Y ont la signification indiquée, en ce que l'on fait réagir celle-ci avec un composé carbonylé de formule III

$R^1$-CO-$R^2$ III

où $R^1$ et $R^2$ ont la signification indiquée, en éliminant l'eau, pour former un composé bicyclique de formule IV

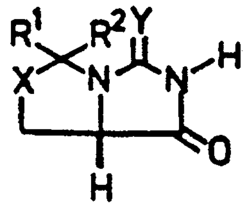

IV

où $R^1$, $R^2$, X et Y ont la signification indiquée, l'atome d'azote secondaire comportant un groupe protecteur $R^3$ ayant la signification indiquée,

ou en ce que l'on fait réagir la L-cystéine ou la L-sérine avec un composé carbonylé de formule III, en éliminant l'eau, pour former une azolidine V

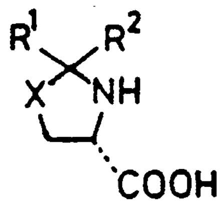

V

où $R^1$, $R^2$ et X ont la signification indiquée, en ce que l'on fait réagir celle-ci avec un cyanate ou un thiocyanate d'un métal alcalin ou alcalino-terreux pour former un composé de formule IV et en ce que l'on procède ensuite comme indiqué précédemment,

ou en ce que l'on fait réagir le composé V avec un organoisocyanate ou un organoisothiocyanate de formule VI

$R^3$-N=C=Y VI

où $R^3$ et Y ont la signification indiquée

ou en ce que l'on fait réagir la L-cystéine avec un cyanate ou un thiocyanate alcalin ou alcalino-terreux pour former une bishydantoïne de formule VII

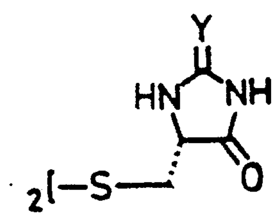

VII

où Y représente O ou S,

ou en ce que l'on transforme celle-ci par un agent réducteur en une hydantoïne de formule II et en ce que l'on fait réagir ensuite celle-ci comme indiqué précédemment ou en ce que l'on munit l'atome d'azote se trouvant en position 3 d'un groupe protecteur $R^3$ ayant la signification indiquée et en ce que l'on fait réagir la bishydantoïne obtenue ce formule VIII

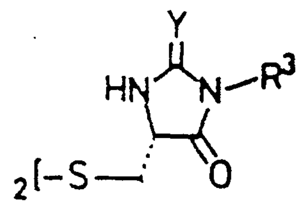

VIII

ou $R^3$ et Y ont la signification indiquée, après clivage avec un agent réducteur, avec un composé carbonylé de formule III,

ou en ce que l'on fait réagir la L-cystine avec un organoisocyanate cu un organoisothiocyanate ayant la formule VI indiquée pour former une bishydantoïne de formule VIII et en ce que l'on procède ensuite comme indiqué précédemment.

**3.** Utilisation des hydantoïnes de formule I selon la revendication 1 pour la préparation de la D-(+)-biotine.